Europäisches Patentamt

European Patent Office

Office européen des brevets

(19)

(11) Veröffentlichungsnummer: **0 478 827 B1**

(12) **EUROPÄISCHE PATENTSCHRIFT**

(45) Veröffentlichungstag der Patentschrift: **06.04.94**

(51) Int. Cl.5: **C12N 9/74**, C12N 9/96, C12Q 1/56

(21) Anmeldenummer: **90118976.1**

(22) Anmeldetag: **04.10.90**

(54) **Stabilisiertes Thrombin, seine Herstellung und seine Verwendung als Thrombinzeitreagens.**

(43) Veröffentlichungstag der Anmeldung:
**08.04.92 Patentblatt 92/15**

(45) Bekanntmachung des Hinweises auf die
Patenterteilung:
**06.04.94 Patentblatt 94/14**

(84) Benannte Vertragsstaaten:
**DE**

(56) Entgegenhaltungen:
**EP-A- 0 123 304**
**EP-A- 0 244 771**
**DD-A- 150 544**
**DE-A- 3 536 903**

**6218183 EMBASE 86213246; Z.S. LATALLO et
al.: "Reaction of thrombins with human antithrombin III. I. Enzyme activity losses unrelated to the inhibitory reaction and their prevention"**

(73) Patentinhaber: **KALLIES IMPORT-EXPORT VER-
TRIEB GMBH**
**Götzingerstrasse 17**
**D-01855 Sebnitz(DE)**

(72) Erfinder: **Töpfer, Gottfried**
**Am Feierabendheim 26**
**O-8900 Görlitz(DE)**
Erfinder: **Friedel, Gisela**
**J.-Fucik-Strasse 43**
**O-8902 Görlitz(DE)**
Erfinder: **Seifert, Andreas**
**Th.-Körner-Strasse 5**
**O-8900 Görlitz(DE)**
Erfinder: **Funke, Udo**
**Rietsc helstrasse 20**
**O-8800 Zittau(DE)**
Erfinder: **Labus, Dieter**
**Ostring 64**
**O-8909 Görlitz(DE)**
Erfinder: **Lutze, Gerd**
**Schreiberstrasse 6**
**O-3090 Magdeburg(DE)**
Erfinder: **Schmidt, Lothar**
**Martin-Opitz-Strasse 1**
**O-8900 Görlitz(DE)**
Erfinder: **Dornheim, Günter**
**Karl-Marx-Strasse 51**
**O-6018 Suhl(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

EP 0 478 827 B1

(74) Vertreter: **Patentanwälte Beetz - Timpe - Siegfried Schmitt-Fumian - Mayr**
**Steinsdorfstrasse 10**
**D-80538 München (DE)**

## Beschreibung

Die Erfindung betrifft ein stabilisiertes Thrombin als Reagens zur Bestimmung der Thrombinzeit, ein Verfahren zu seiner Herstellung und seine Verwendung.

Thrombin (Gerinnungsfaktor IIa) hat eine große Bedeutung für hämostaseologische Untersuchungen. Die Instabilität von Thrombin besonders in Lösung beeinträchtigt aber die Ergebnisse analytischer Methoden und damit deren Anwendbarkeit bei der Klärung diagnostischer und anderer Fragestellungen.

Es ist bekannt, daß die Instabilität von Thrombin bei der Thrombinzeitbestimmung (z.B. nach A.B.(D.L.)-DDR) einerseits besondere Festlegungen erfordert, welche die Herstellung, Einstellung und Haltbarkeit der Thrombin-RL[+] betreffen, andererseits wird der Test durch das Fehlen eines stabilen und standardisierten Reagens arbeitsaufwendig, und die Verwertbarkeit der Ergebnisse für bestimmte diagnostische Fragestellungen ist eingeschränkt bzw. wird durch die mangelnde Genauigkeit und Zuverlässigkeit der Methode beeinträchtigt.

So muß z.B. die Thrombin-RL für die Bestimmung der Thrombinzeit des Plasmas (z.B. nach A.B.(D.L.)-DDR-83) für jede Untersuchungsserie in ihrer Aktivität neu mit Normalplasma auf 17,5 ± 0,5 s eingestellt werden.

Die Haltbarkeit von Thrombin in Polyethylengefäßen bei 37 °C wird mit 1 h angegeben. Genauigkeit und Richtigkeit der Methode lassen daher zu wünschen übrig. Diese Methode ist ferner nicht zur Überwachung der Heparintherapie geeignet.

Aus DD-A-158857 ist ein Thrombinstabilisator für Laboratoriumsdiagnostika bekannt, der auf der synergistischen Wirkung von Proteaseinhibitoren (Aprotinin), einem Chelatbildner, vorzugsweise Ethylendiamintetraessigsäure (EDTA), und Rinderserumalbumin beruht. Als Substrat für das stabilisierte Thrombin wird dabei das chromogene Substrat Tos-Gly-Pro-Arg-pNa verwendet.

Dieser Stabilisator ist für die Thrombinzeitbestimmung (Substrat Fibrinogen) nicht verwendbar, da die Chelatbildner die Calciumionen des Plasmas weitgehend binden, wodurch es zu einer Polymerisationshemmung bei der Fibrinogen-Fibrin-Umwandlung kommt. Infolgedessen werden 100-fach höhere Thrombin-Konzentrationen benötigt, um eine Gerinnung von Plasma zu erreichen. Der Test ist jedoch in dieser Form nicht mehr für die Thrombinzeitbestimmung hinsichtlich der diagnostischen Fragestellung geeignet, ob eine Antithrombinwirkung bzw. eine Polymerisationshemmung oder ein Fibrinogenmangel bzw. eine Dysfibrinogenämie vorliegen. Werden die Chelatbildnerkomponente und das Aprotinin weggelassen (M. Miller-Andersson et al.: Preparation and Stability of a Highly Purified Human Thrombin Standard, Thromb. Res 20 (1980) 109-122), so ergibt sich bei hochgereinigtem Thrombin allein durch die Albuminkomponente schon ein thrombinstabilisierender Effekt; die erreichte Stabilität ist jedoch nicht ausreichend. Das Verfahren ist außerdem kostenaufwendig, bedingt durch den relativ hohen Preis des Albumins.

Gemäß DD-A-150544 wird Gelafusal®, eine Lösung partiell abgebauter, durch Hydrolyse von Collagen erhaltener Gelatine, zur Stabilisierung von Streptokinase eingesetzt. Allerdings ist in dieser Druckschrift nur eine Stabilitätserhöhung der Lyophilisate beschrieben, und die vorgeschlagenen Gelafusalkonzentrationen sind für eine Stabilisierung des Thrombins nicht wirksam.

HEPES (4-(2-Hydroxyethyl)-1-piperazinethansulfonsäure) ist ferner als hervorragender zwitterionischer Puffer zur Stabilisierung von Plasma beschrieben (G. Töpfer et al.: Beitrag zur Kontrolle der Qualität von Gerinnungsanalysen, 1. Mitt.; Zbl. Pharm. 123 (1984) 697-701), jedoch ist ein thrombinstabilisierender Effekt bisher unbekannt.

Infolge der Abgabe von $CO_2$ aus dem Plasma kommt es innerhalb von 2 bis 3 h nach der Blutentnahme (z.B. Citratblutgewinnung nach A.B.(D.L.)-DDR) zu einer pH-Wert-Erhöhung des Plasmas, die in Abhängigkeit des Verhältnisses von Oberfläche zu Volumen unterschiedlich hoch ausfällt.

Diese Veränderung des pH-Werts kann das Ergebnis der Thrombinzeitbestimmung in einem solchen Umfang beeinflussen, daß vollkommen unbrauchbare Resultate erhalten werden (vgl. Tab. 3).

Der Erfindung liegt die Aufgabe zugrunde, ein als Thrombinzeitreagens geeignetes stabilisiertes Thrombin und seine Herstellung und Verwendung anzugeben, womit die bestehenden, auf der Instabilität des Thrombins beruhenden Schwierigkeiten bei der Bestimmung der Thrombinzeit überwunden werden sollen.

Neben der Gewährleistung einer ausreichenden Lagerungsstabilität soll das Thrombinzeitreagens ohne Aktivitätsverlust lyophilisiert werden können und in der gefriergetrockneten Form stabil sein. Weiterhin sollen die Genauigkeit und Zuverlässigkeit der Thrombinzeitbestimmung verbessert und ihre Anwendungsfreundlichkeit erhöht werden.

Das stabilisierte Thrombin sollte ferner auch eine Thrombinzeitbestimmung ohne Beeinflussung durch Heparin gestatten.

Die Aufgabe wird anspruchsgemäß gelöst. Die abhängigen Ansprüche betreffen vorteilhafte Ausführungsformen.

Das erfindungsgemäße stabilisierte Thrombin enthält, neben dem Thrombin, drei Stabilisatoren:

Stabilisator I : HEPES-Puffer,

Stabilisator II : Thiomersal und

Stabilisator III: durch partielle Hydrolyse von Collagen erhaltene Gelatine.

Das stabilisierte Thrombin liegt vorteilhaft in Form einer isotonischen Salzlösung, insbesondere einer isotonischen Kochsalzlösung, vor.

Das erfindungsgemäß stabilisierte Thrombinzeitreagens enthält, sofern es in Lösung vorliegt, vorteilhaft als Pufferkomponente 0,03 bis 0,06 mol/l HEPES, vorzugsweise 0,05 mol/l, vom pH 7,55 (gemessen bei 20 °C). Dieser pH-Wert entspricht dem pKA, wodurch eine hohe Pufferkapazität erreicht wird (Tab. 3).

Selbst ein völlig von $CO_2$ entgastes Plasma (pH = 8,6) bleibt in seiner Thrombinzeit unbeeinflußt, da im Reagens-Plasma-Gemisch der pH-Wert unter 7,8 liegt.

Die Erfindungskonzeption beruht auf der überraschenden Feststellung, daß HEPES-Puffer als Thrombin-Stabilisator wirkt (Tab. 1) (Stabilisator I).

Das Thiomersal (Na-Salz der Ethylmercurithiosalicylsäure) (Stabilisator II) dient dazu, in der Thrombinlösung ein Wachstum von Mikroorganismen zu unterbinden.

Überraschenderweise wurde aber auch gefunden, daß Thiomersal in der angegebenen Konzentration einen thrombinstabilisierenden Effekt ergibt und mit dem HEPES-Puffer synergistisch wirkt (Tab. 1). Es wurde gefunden, daß 25 bis 40 ml Gelafusal (Serumwerk Bernburg) und vorzugsweise 33 ml je Liter Thrombinzeitreagens zu einer weiteren Thrombinstabilisierung führen (Stabilisator III), wobei Gelafusal 35 g durch partiellen Collagenabbau erhaltene Gelatine (mittlere Molekülmasse M = 35 kD) und 8,0 g Natriumchlorid pro Liter enthält.

Die vorzugsweise eingesetzten 33 ml Gelafusal entsprechen einer Konzentration von 1,16 g Gelatine/l Thrombinzeitreagens.

Zur Verwendung des Thrombinzeitreagens hat das erfindungsgemäße stabilisierte Thrombin vorteilhaft folgende Zusammensetzung pro 2500 NIH-Einheiten Thrombin in isotonischer Kochsalzlösung, vorzugsweise 0,154 mol/l, je Liter:

Stabilisator I : 26,4 bis 52,8 ml und vorzugsweise 44 ml HEPES-Pufferlösung einer Konzentration von 1,15 mol/l,

Stabilisator II : 250 bis 750 mg und vorzugsweise 500 mg Thiomersal und

Stabilisator III: 25 bis 40 ml und vorzugsweise 33 ml Gelafusal (erhalten durch partielle Hydrolyse von Collagen) mit einem Gehalt von 35 g partiell hydrolysierter Gelatine und 8,0 g NaCl pro Liter.

Das erfindungsgemäße stabilisierte Thrombin kann ferner auch als Stabilisator IV Polybren enthalten, vorzugsweise in einer Menge von 10 ml Polybrenlösung einer Konzentration von 1,1 mg/ml pro Liter.

Das stabilisierte Thrombin wird gemäß der Erfindung so hergestellt, daß zunächst das Thrombin, vorteilhaft handelsübliches, nicht besonders gereinigtes Thrombin, in isotonischer Salzlösung, vorteilhaft Kochsalzlösung, (0,154 mol NaCl/l) gelöst wird, worauf dann die Stabilisatoren I bis III und gegebenenfalls auch der Stabilisator IV zugegeben werden.

Das stabilisierte Thrombin kann vorteilhaft auch so hergestellt werden, daß die erforderliche Menge Thrombin in einer isotonischen Salzlösung, insbesondere Kochsalzlösung, die bereits die Stabilisatoren I bis III und im Falle eines heparinunempfindlichen Reagens auch den Stabilisator IV enthält, gelöst wird.

Dieses Gemisch kann zur längeren Lagerung lyophilisiert werden. Wiederaufgelöstes Lyophilisat zeichnet sich durch gleichbleibende, reproduzierbare Thrombinaktivität aus.

Lösungen des stabilisierten Thrombins, die direkt durch Mischen der Komponenten oder durch Auflösen eines Lyophilisats hergestellt sind, können ohne Aktivitätsverlust wiederholte Male, vorteilhaft durch Abkühlen auf -20 °C, eingefroren und, vorteilhaft durch Erwärmen auf +20 °C, wiederaufgetaut und verwendet werden.

Das erfindungsgemäße Verfahren zur Thrombinzeitbestimmung ist dadurch gekennzeichnet, daß stabilisiertes Thrombin gemäß der Erfindung eingesetzt wird. Das Thrombinzeitreagens wird günstigerweise mit Normalplasma auf die gewünschte Thrombinaktivität eingestellt, insbesondere so, daß Normalplasma innerhalb von 17 ± 0,2 s (Mittelwert aus 10 Bestimmungen) gerinnt.

Durch die spezielle Stabilisierung mit den Stabilisatoren I, II und III wird das Thrombin derart stabilisiert, daß es mehreren Gefrier-Auftau-Zyklen unterzogen und auch lyophilisiert werden kann, wobei die Lyophilisate eine hohe Lagerungsstabilität aufweisen. Mit dem stabilisierten Thrombin als Thrombinzeitreagens läßt sich die Thrombinzeit diagnostisch mit oder ohne Heparinabhängigkeit bestimmen, so daß es auch zur Überwachung der Heparintherapie im niedrigdosierten Bereich herangezogen werden kann.

Das erfindungsgemäße stabilisierte Thrombin kann also ohne Aktivitätsverlust in eingefrorenem wie auch in lyophilisiertem Zustand gelagert werden.

Das Thrombinzeitreagens zeichnet sich durch eine verbesserte Gebrauchsdauer im gelösten, flüssigen und im tiefgefrorenen Zustand bei Temperaturen zwischen vorzugsweise -20 °C und +20 °C, aber auch bei der Meßtemperatur von 37 °C aus, wobei mehrere Gefrier-Auftau-Zyklen mit dem gleichen Reagens möglich sind. Weiterhin werden mit der vorgeschlagenen Zusammensetzung des Thrombinzeitreagens die Genauigkeit und Zuverlässigkeit der Thrombinzeitbestimmung verbessert, und es wird die Möglichkeit geschaffen, den bekannten Einfluß von Heparin auf das Ergebnis der Thrombinzeitbestimmung wahlweise auszuschalten oder das Ergebnis für die diagnostische Fragestellung der Beurteilung der Heparinwirkung im niedrigdosierten Bereich bei der Heparintherapie gezielt zu nutzen.

Das oben angegebene erfindungsgemäße Verfahren zur Stabilisierung von Thrombin als Thrombinzeitreagens beinhaltet vorzugsweise das Auflösen von etwa 2500 NIH-Einheiten Thrombin (beispielsweise Thrombin für Testzwecke, Arzneimittelwerk Dresden GmbH) in einem Gemisch aus isotonischer Kochsalzlösung (0,154 mol/l NaCl), der die Stabilisatoren I (HEPES-Puffer, II (Thiomersal) und III (Gelafusal) im angegebenen Konzentrationsbereich unter Rühren zugegeben wurden, so daß das Gesamtvolumen 1,00 l beträgt. Dieses Gemisch wird im folgenden als Stabilisator I-III bezeichnet.

Die hergestellte Thrombinlösung wird in silikonisierten Glasgefäßen (z.B. Silikonisierung nach A.B.(D.L.)-DDR) aufbewahrt. Die stabilisierte Thrombinlösung wird nach A.B.(D.L.)-DDR mit einem Normalplasma als Substrat durch Zugabe von Thrombin, das mit NIH-E/ml im Stabilisator I-III gelöst ist, bzw. durch Zugabe von Stabilisator I-III allein so eingestellt, daß bei der Thrombinzeit (TZ) nach A.B.(D.L.)-DDR das Normalplasma innerhalb von 17,0 ± 0,2 s gerinnt.

Es wurde festgestellt, daß bei der Durchführung der Thrombinzeitbestimmung mit dem erfindungsgemäßen Thrombinzeitreagens die Methode heparinempfindlich ist; bei Verwendung von Thrombin für Testzwecke (Arzneimittelwerk Dresden GmbH) zeigt die TZ Verlängerungen bei ≥ 0,025 IE Heparin/ml (Tab. 4).

Setzt man dieser Thrombinlösung (Stabilisatoren I-III) Polybren (Hexadimethrinbromid, Poly(1,5-Dimethyl-1,5-diazoundecamethylenmethobromid), Copolymer aus N,N,N',N'-Tetramethyl-1,6-hexandiamin und 1,3-Dibrompropan, Heparin-Antagonist) (Stabilisator IV) in einer Konzentration von 0,11 g pro Liter Thrombinzeitreagens zu, so kommt es zu einer weiteren Stabilitätserhöhung des Thrombins (Tab. 1) (als Stabilisator I-IV bezeichnet).

Die Einstellung der Thrombinaktivität des Thrombinzeitreagens erfolgt wie beschrieben mit Normalplasma, wobei jedoch vorteilhaft Thrombin (100 NIH-E/ml) in dem Gemisch der Stabilisatoren I-IV gelöst und diese Stabilisatoren selbst zur Verdünnung des Thrombinzeitreagens eingesetzt werden.

Es wurde gefunden, daß Polybren in der angegebenen Konzentration von 0,11 g/l 2 bis 3 IU Heparin/ml inhibiert, so daß eine Thrombinzeitbestimmung unabhängig von Heparin möglich ist (Tab. 4).

Es wurde ferner festgestellt, daß die Empfindlichkeit der Thrombinzeit auf FDP, Dysfibrinogenämie bzw. Fibrinogenmangel unbeeinflußt bleibt. Demgemäß ist aus den Ergebnissen der Thrombinzeitbestimmung mit und ohne Polybren (Stabilisator I-IV, Stabilisator I-III) eine Einschätzung der Heparinwirkung bei einer Heparintherapie im niedrigen Dosierungsbereich möglich (Tab. 4).

Ein wesentlicher Vorteil des neuen Verfahrens ist die Tatsache, daß die Thrombin-RL$^+$-Reagenslösung nicht mehr für jede Untersuchungsserie neu in ihrer Aktivität eingestellt werden muß. Dadurch wird das Verfahren wesentlich anwendungsfreundlicher und präziser, Vorteile, die sich besonders im Bereitschaftsdienst und Nachtdienst zeigen. (Einzelfehler bei der Aktivitätseinstellung entfallen, vgl. Tab. 6).

Es wurde festgestellt, daß sowohl das Thrombinzeitreagens mit den Stabilisatoren I-III als auch das mit den Stabilisatoren I-IV ohne Aktivitätsverlust lyophilisiert werden kann (Tab. 2) und daß das Lyophilisat während der Lagerung stabil ist (Tab. 5). Ferner ergab sich, daß bei der Lyophilisation die Verwendung silikonisierter Glasgefäße zweckmäßig ist. Damit eröffnet die Erfindungskonzeption auch die Möglichkeit, Thrombinzeitreagentien industriell herzustellen und in standardisierter Form, d.h. auf die geforderte Thrombinaktivität eingestellt, in den Verkehr zu bringen.

Untersuchungen ergaben, daß die Thrombinzeitreagentien bis zu 10 Mal einem Gefrier-Auftau-Zyklus ausgesetzt werden können.

## Anwendungsbeispiel

Herstellung des Thrombinzeitreagens und Einstellung auf die gewünschte Thrombinaktivität erfolgten wie bereits beschrieben.

**Thrombinzeitreagens mit den Stabilisatoren I-III**

Etwa 2500 NIH-Einheiten Thrombin (Thrombin für Testzwecke, Arzneimittelwerk Dresden GmbH) werden in einem Gemisch aus 923 ml isotonischer Kochsalzlösung (0,154 mol/l NaCl) gelöst, der die folgenden Komponenten unter Rühren zugesetzt wurden:

Stabilisator I : 44 ml HEPES-Puffer vom pH = pKA = 7,55 mit einer Konzentration von 1,15 mol/l;

Stabilisator II : 500 mg Thiomersal;

Stabilisator III: 33 ml Gelafusal.

**Thrombinzeitreagens mit den Stabilisatoren I-IV**

Zu einem Liter des Gemischs mit den Stabilisatoren I-III setzt man als Stabilisator IV 10 ml einer Lösung von Polybren (1,1 g/l) zu und löst darin 2500 NIH-Einheiten Thrombin.

In den nachfolgenden Tabellen wird ein Überblick über die stabilisierenden Eigenschaften, die Puffereigenschaften und die Genauigkeitssteigerung der Stabilisatoren I-IV gegeben.

## Tabelle 1

### Synergistische stabilisierende Wirkung von Stabilisator I-IV

| Stabilisator | Stabilität in Tagen (d) | |
| --- | --- | --- |
| | bei +4 °C | bei +20 °C |
| Thrombin mit isotonischer Koch-salzlösung | | |
| (ohne Stabilisator) | 3 | 1 |
| mit HEPES (I) | 8 | 2 |
| Thiomersal (II) | 8 | 1 |
| Gelafusal (III) | 15 | 3 |
| Polybren (IV) | 15 | 15 |
| HEPES + Thiomersal | 15 | 3 |
| HEPES + Gelafusal | 15 | 3 |
| HEPES + Polybren | 15 | 3 |
| Thiomersal + Polybren | 15 | 15 |
| Gelafusal + Polybren | 15 | 15 |
| HEPES + Thiomersal + Gelafusal (I-III) | 150 | 15 |
| HEPES + Thiomersal + Polybren | 15 | 15 |
| Thiomersal + Gelafusal + Polybren | 15 | 15 |
| HEPES + Gelafusal + Polybren | 150 | 15 |
| HEPES + Thiomersal + Gelafusal + Polybren | 180 | 15 |
| zum Vergleich: | | |
| Rinderserumalbumin (1 %) | 5 | 2 |

7

Tabelle 2

| Stabilität der erfindungsgemäßen Stabilisatorkombinationen nach Einfrier-Auftau-Zyklen bzw. nach Lyophilisation | | |
|---|---|---|
| Reagens | Anzahl der Einfrier-Auftau-Zyklen ohne Aktivitätsverlust | TZ vor/nach Lyophilisation (s) |
| Thrombin RL* nach A.B. (ohne Stabilisatoren) | O (Aktivität sinkt nach 1 Zyklus auf rund 50 %) | 17,0/60,2 |
| mit Stabilisator I-III | 5 | 16,8/16,8 |
| mit Stabilisator I-IV | 10 | 17,0/17,1 |

Tabelle 3

| pH-Wert-Abhängigkeit der Thrombinzeit nach A.B.(D.L.)-DDR und der mit HEPES stabilisierten Thrombinzeitvariante gemäß der Erfindung | | |
|---|---|---|
| pH-Wert des Plasmas | TZ nach A.B. (s) | TZ stabilisiert mit HEPES (s) |
| 7,40 | 26,8 | 23,8 |
| 7,80 | 28,2 | 24,0 |
| 8,00 | 28,4 | 23,9 |
| 8,20 | 37,0 | 24,2 |
| 8,40 | 43,0 | 24,3 |
| 8,60 | größer 180 | 24,5 |

Tabelle 4

| Einfluß von Heparin auf die TZ (s) mit und ohne Polybren im Reagens | | | | | | | | | |
|---|---|---|---|---|---|---|---|---|---|
| Heparinkonzentration (E/ml) | 0 | 0,025 | 0,05 | 0,075 | 0,1 | 0,5 | 1,0 | 2,0 | 3,0 | 5,0 |
| Stabilisatoren I -III | 18,0 | 20,2 | 32,5 | 64,8 | 194 | >300 | >300 | >300 | >300 | >300 |
| Stabilisatoren I -IV | 17,8 | 17,9 | 18,0 | 17,8 | 18,0 | 17,9 | 18,2 | 18,1 | 18,3 | 27,8 |

Tabelle 5

| Stabilität von lyophilisiertem Thrombinreagens | | | | | |
|---|---|---|---|---|---|
| Stabilisator | Anzahl der Einfrier-Auftau-Zyklen ohne Stabilitätseinbuße | Stabilität des Lyophilisats bei +4 °C | Stabilität des gelösten Lyophilisats bei | | |
| | | | +4 °C | +20 °C | +37 °C |
| | | | aufbewahrt zu je 2 ml | | |
| Stabilisator I -III | 3 | >8 Monate | 7 d | 3 d | 2 h |
| Stabilisator I -IV | 5 | >8 Monate | 7 d | 4 d | 2 h |

## Tabelle 6

### Abhängigkeit der zeitabhängigen Genauigkeit von der Verfahrensweise bzw. vom Stabilisator der TZ-Bestimmung

1. Nach A.B. (D.L.)DDR   (Thrombinaktivität ist für jede Serie mit Normalplasma erneut einzustellen.)

2. Stabilisator I –III        25 d bei + 4 °C stabil

3. Stabilisator J –IV        180 d bei + 4 °C stabil

4. Lyophilisate mit Stabilisator I –III   (täglich neu auflösen)

5. Lyophilisate mit Stabilisator I –IV   (täglich neu auflösen)

| zeitabhängige Genauigkeit S (%) | Prüfmaterial = Kontrollplasma mit TZ = 19,0 s | | | | |
|---|---|---|---|---|---|
| | 1. | 2. | 3. | 4. | 5. |
| | 5,2 | 2,0 | 1,5 | 2,8 | 2,3 |

Die obigen experimentellen Ergebnisse zeigen die überraschende, starke synergistische Wirkung der Stabilisatorkomponenten und die große Stabilität des erfindungsgemäß stabilisierten Thrombins.

**Patentansprüche**

1. Stabilisiertes Thrombin, insbesondere zur Verwendung als Thrombinzeitreagens, auf der Basis von Thrombin und eines Stabilisators,
**gekennzeichnet durch** ein Stabilisatorgemisch aus
Stabilisator I :        HEPES-Puffer,
Stabilisator II :        Thiomersal,
Stabilisator III:        durch partielle Hydrolyse von Collagen erhaltene Gelatine.

2. Stabilisiertes Thrombin nach Anspruch 1, gekennzeichnet durch ein Stabilisatorgemisch, das ferner als Stabilisator IV Polybren enthält.

9

**3.** Stabilisiertes Thrombin nach den Ansprüchen 1 und 2, dadurch gekennzeichnet, daß es in Form einer isotonischen Salzlösung, vorzugsweise einer isotonischen Kochsalzlösung (Konzentration 0,154 mol/l), vorliegt.

**4.** Stabilisiertes Thrombin nach den Ansprüchen 1 bis 3, gekennzeichnet durch
- 0,03 bis 0,06 mol/l und vorzugsweise 0,05 mol/l Stabilisator I,
- 250 bis 750 mg/l und vorzugsweise 500 mg/l Stabilisator II
  und
- 25 bis 40 ml und vorzugsweise 33 ml Stabilisator III, der einen Gehalt von 35 g Gelatine und 8,0 g NaCl pro Liter aufweist,
  sowie gegebenenfalls
- 0,11 g/l Polybren.

**5.** Stabilisiertes Thrombin nach den Ansprüchen 1 bis 4, dadurch gekennzeichnet, daß es in isotonischer Kochsalzlösung pro Liter enthält:
- etwa 2500 NIH-Einheiten Thrombin, vorzugsweise handelsübliches, nicht besonders gereinigtes Thrombin,
- 26,4 bis 52,8 ml und vorzugsweise 44 ml HEPES-Puffer vom $pH = pKA = 7,55$, einer Konzentration von 1,15 mol/l (Stabilisator I),
- 250 bis 750 mg und vorzugsweise 500 mg Thiomersal (Stabilisator II)
  und
- 25 bis 40 und vorzugsweise 33 ml einer Gelatinelösung mit einem Gehalt von 35 g Gelatine und 8,0 g NaCl pro Liter
  sowie gegebenenfalls
- 0,11 g Polybren.

**6.** Stabilisiertes Thrombin nach den Ansprüchen 1 bis 5, dadurch gekennzeichnet, daß es so eingestellt ist, daß Normalplasma innerhalb einer mittleren Zeit von 17 ± 0,2 s gerinnt.

**7.** Stabilisiertes Thrombin nach den Ansprüchen 1 bis 6, dadurch gekennzeichnet, daß es in trockener, lyophilisierter Form, vorzugsweise in Dosiereinheiten mit eingestellter Thrombinaktivität, oder in Form einer flüssigen oder eingefrorenen Lösung, vorzugsweise mit eingestellter Thombinaktivität, vorliegt.

**8.** Verfahren zur Herstellung des stabilisierten Thrombins nach den Ansprüchen 1 bis 7, gekennzeichnet durch
(A)
1. Herstellung einer vorzugsweise isotonischen Salzlösung, bevorzugt einer isotonischen Kochsalzlösung, der Stabilisatoren I, II und III und im Falle der Herstellung eines heparinunempfindlichen Reagens der Stabilisatoren I, II, III und IV mit den erforderlichen Konzentrationen und
2. Lösung der erforderlichen Menge Thrombin, vorteilhaft von handelsüblichem, nicht besonders gereinigtem Thrombin, im Stabilisatorgemisch
sowie gegebenenfalls
3. Einfrieren der erhaltenen stabilisierten Thrombinlösung, vorteilhaft bei etwa -20 °C,
oder
(B)
1. Herstellung einer Salzlösung, bevorzugt einer Kochsalzlösung, von Thrombin
und
2. Zusatz der Stabilisatoren I, II und III und im Falle der Herstellung eines heparinunempfindlichen Reagens der Stabilisatoren I, II, III und IV in den jeweils erforderlichen Mengen, vorzugsweise unter Einstellung einer isotonischen Lösung,
sowie gegebenenfalls
3. Einfrieren der erhaltenen stabilisierten Thrombinlösung, vorteilhaft bei etwa -20 °C,
oder
(C)
1. Lyophilisierung einer gemäß den Verfahrensvarianten A oder B erhaltenen stabilisierten Thrombinlösung
und

2. Auflösen des Lyophilisats zur Verwendung in Wasser oder einer Salzlösung sowie gegebenenfalls

3. Einfrieren der erhaltenen stabilisierten Thrombinlösung, vorteilhaft bei etwa -20 °C,

oder

(D)

1. Auftauen einer gemäß den Verfahrensvarianten A, B oder C erhaltenen und, vorteilhaft bei etwa -20 °C, eingefrorenen stabilisierten Thrombinlösung, vorzugsweise bei etwa 20 °C, und gegebenenfalls

2. Wiedereinfrieren der stabilisierten Thrombinlösung, vorteilhaft bei etwa -20 °C, sowie gegebenenfalls

3. Wiederauftauen der (wieder)eingefrorenen stabilisierten Thrombinlösung.

9. Verfahren zur Thrombinzeitbestimmung durch Gerinnung von Plasma, vorzugsweise von Normalplasma, mit stabilisiertem Thromin, gekennzeichnet durch Verwendung des stabilisierten Thrombins nach den Ansprüchen 1 bis 7 bzw. eines nach einer der Verfahrensvarianten gemäß Anspruch 8 erhältlichen stabilisierten Thrombins als Thrombinzeitreagens.

10. Diagnoseset, gekennzeichnet durch ein stabilisiertes Thrombin nach Anspruch 7 und gegebenenfalls Wasser oder eine wäßrige Lösung zum Lösen des Thrombins.

## Claims

1. Stabilized thrombine, particularly for use as a clotting-time agent, on the basis of thrombine and a stabilizer

   **characterized** by a stabilizer mixture composed of

   a stabilizer I : HEPES-buffer

   a stabilizer II : thiomersal

   a stabilizer III: gelatine obtained by partial hydroysis of collagen.

2. Stabilized thrombine according to claim 1, characterized by a stabilizer mixture additionally containing as stabilizer IV polybren.

3. Stabilized thrombine according to claims 1 and 2, characterized in that it exists in the form of an isotonic salt solution, preferably an isotonic common salt solution (concentration 0.154 mol/l).

4. Stabilized thrombine according to claims 1 to 3, characterized by
   - 0.03 to 0.06 mol/l, preferably 0.05 mol/l, of stabilizer I,
   - 250 to 750 mg/l, preferably 500 mg/l, of stabilizer II, and
   - 25 to 40 ml, preferably 33 ml, of stabilizer III having a content of 35 g gelatine and 8.0 g NaCl per liter, and optionally
   - 0.11 g/l of polybren.

5. Stabilized thrombine according to claims 1 to 4, characterized in that it contains per liter in an isotonic common salt solution:
   - approximately 2500 NIH-units of thrombine, preferably commercial, not particularly purified thrombine,
   - 26.4 to 52.8 ml, preferably 44 ml, of HEPES-buffer of $pH = pKA = 7.55$ at a concentration of 1.15 mol/l (stabilizer I),
   - 250 to 750 mg, preferably 500 mg, of thiomersal (stabilizer II), and
   - 25 to 40 mg, preferably 33 mg, of a gelatine solution containing per liter 35 g gelatine and 8.0 g NaCl, and optionally
   - 0.11 g of polybren.

6. Stabilized thrombine according to claims 1 to 5, characterized in that it is so adjusted that normal plasma clots within a mean time of 17 ± 0.2 s.

7. Stabilized thrombine according to claims 1 to 6, characterized in that it exists in dry lyophilized form, preferably in metered units with adjusted thrombine activity, or in the form of a liquid or frozen solution, preferably with adjusted thrombine activity.

8. Process for preparing the stabilized thrombine according to claims 1 to 7, characterized by

(A)

1. preparing a preferably isotonic salt solution, preferably an isotonic common salt solution, of the stabilizers I, II and III, and, in of case of preparing a heparin-insensitive reactant, of the stabilizers I, II, III and IV at the required concentrations, and

2. dissolving the required amount of thrombine, preferably of commercial, not particularly purified thrombine, in the stabilizer mixture, and optionally

3. freezing the resulting stabilized thrombine solution, suitably at approximately -20 °C, or

(B)

1. preparing a salt solution, preferably a common salt solution, of thrombine, and

2. adding the stabilizers I, II and III, and, in case of preparing a heparin-insensitive reactant, the stabilizers I, II, III and IV in the amounts required in each instance, preferably with adjusting an isotonic solution, and optionally

3. freezing the resulting stabilized thrombine solution, suitably at approximately -20 °C, or

(C)

1. lyophilizing a stabilized thrombine solution obtained according process variants A or B, and

2. dissolving the lyophilisate for use in water or a salt solution, and optionally

3. freezing the resulting stabilized thrombine solution, suitably at approximately -20 °C, or

(D)

1. thawing, suitably at approximately 20 °C, a stabilized thrombine solution obtained according to process variants A, B or C, and frozen suitably at -20 °C, and optionally

2. refreezing the stabilized thrombine solution, suitably at approximately -20 °C, and optionally

3. rethawing the (re-)frozen stabilized thrombine solution.

9. Process for determining the clotting-time by clotting of plasma, preferably standard plasma, with the use of stabilized thrombine, characterized by using as clotting-time agent the stabilized thrombine according to claims 1 to 7, and/or a stablized thrombine obtainable according to one of the process variants of claim 8.

10. Diagnostic set characterized by a stabilized thrombine according to claim 7, and optionally water, or an aqueous solution, for dissolving the thrombine.

**Revendications**

1. Thrombine stabilisée, destinée en particulier à être utilisée comme réactif pour le temps de thrombine, à base de thrombine et d'un stabilisant, caractérisée par un mélange de stabilisants constitué par :
   un stabilisant I : tampon HEPES
   un stabilisant II : thiomersal
   un stabilisant III : gélatine obtenue par hydrolyse partielle du collagène.

2. Thrombine stabilisée selon la revendication 1, caractérisée par un mélange de stabilisants qui contient en outre du polybrène en tant que stabilisant IV.

3. Thrombine stabilisée selon les revendications 1 et 2, caractérisée en ce qu'elle est sous forme d'une solution saline isotonique, de préférence sous forme d'une solution isotonique de chlorure de sodium (concentration 0,154 mol/l).

4. Thrombine stabilisée selon les revendications 1 à 3, caractérisée par
   - 0,03 à 0,06 mol/l et de préférence 0,05 mol/l de stabilisant I,
   - 250 à 750 mg/l et de préférence 500 mg/l de stabilisant II et
   - 25 à 40 ml et de préférence 33 ml de stabilisant III, qui présente une teneur en gélatine de 35 g et en NaCl de 8,0 g par litre,
        et éventuellement
   - 0,11 g/l de polybrène.

**5.** Thrombine stabilisée selon les revendications 1 à 4, caractérisée en ce qu'elle contient par litre dans une solution isotonique de chlorure de sodium :

- environ 2 500 unités NIH de thrombine, de préférence de thrombine du commerce qui n'est pas particulièrement purifiée,
- 26,4 à 52,8 ml et de préférence 44 ml de tampon HEPES de pH = pKA = 7,55, d'une concentration de 1,15 mol/l (stabilisant I),
- 250 à 750 mg et de préférence 500 mg de thiomersal (stabilisant II) et
- 25 à 40 ml et de préférence 33 ml d'une solution de gélatine d'une teneur en gélatine de 35 g et en NaCl de 8,0 g par litre et éventuellement
- 0,11 g de polybrène.

**6.** Thrombine stabilisée selon les revendications 1 à 5, caractérisée en ce qu'elle est ajustée de telle manière qu'un plasma normal coagule en un temps moyen de 17±0,2 s.

**7.** Thrombine stabilisée selon les revendications 1 à 6, caractérisée en ce qu'elle est sous forme sèche, lyophilisée, de préférence dans des unités de prise à activité de thrombine ajustée, ou sous forme d'une solution liquide ou congelée, de préférence à activité de thrombine ajustée.

**8.** Procédé de préparation de la thrombine stabilisée selon les revendications 1 à 7, caractérisé par

(A)

1. la préparation d'une solution saline de préférence isotonique, de préférence d'une solution isotonique de chlorure de sodium, des stabilisants I,II et III et, dans le cas de la préparation d'un réactif insensible à l'héparine, des stabilisants I, II, III et IV en les concentrations nécessaires et

2. la dissolution dans le mélange de stabilisants de la quantité nécessaire de thrombine, de préférence de thrombine du commerce qui n'est pas particulièrement purifiée et éventuellement

3. la congélation de la solution de thrombine stabilisée obtenue, de préférence à environ -20°C,

ou

(B)

1. la préparation d'une solution saline, de préférence d'une solution de chlorure de sodium, de thrombine et

2. l'addition des stabilisants I, II et III et, dans le cas de la préparation d'un réactif insensible à l'héparine, des stabilisants I, II, III et IV en les quantités nécessaires dans chaque cas, de préférence avec formation d'une solution isotonique, et éventuellement

3. la congélation de la solution de thrombine stabilisée obtenue, de préférence à environ -20°C,

ou

(C)

1. la lyophilisation d'une solution de thrombine stabilisée obtenue selon les variantes de procédé A ou B et

2. la dissolution du lyophilisat pour son utilisation dans l'eau ou dans une solution saline et éventuellement

3. la congélation de la solution de thrombine stabilisée obtenue, de préférence à environ -20°C,

ou

(D)

1. la décongélation de préférence à environ 20°C, d'une solution de thrombine stabilisée obtenue selon les variantes de procédé A, B ou C et congelée de manière avantageuse à environ -20°C, et éventuellement

2. la recongélation de la solution de thrombine stabilisée, de préférence à environ -20°C, et éventuellement

3. la redécongélation de la solution de thrombine stabilisée (re)congelée.

9. Procédé de détermination du temps de thrombine par coagulation d'un plasma, de préférence d'un plasma normal, avec de la thrombine stabilisée, caractérisé par l'utilisation comme réactif pour le temps de thrombine de la thrombine stabilisée selon les revendications 1 à 7 ou d'une thrombine stabilisée qui peut être obtenue par l'une des variantes de procédé selon la revendication 8.

10. Trousse de diagnostic, caractérisée par une thrombine stabilisée selon la revendication 7 et éventuellement de l'eau ou une solution aqueuse pour la dissolution de la thrombine.